# EUROPEAN PATENT SPECIFICATION

(11) **EP 1 588 668 B1**
(45) Date of publication and mention of the grant of the patent: **10.12.2008**
(21) Application number: 05252457.6
(22) Date of filing: 20.04.2005
(51) Int. Cl.: A61B 17/17, A61B 17/15

(54) **Alignment guide for use in femoral head surgery**
Zielgerät zur Operationen des Femurkopfs
Guide d' alignement pour la chirurgie de la tête de fémur

(30) Priority: 20.04.2004 GB 0408792; 02.09.2004 GB 0419494
(43) Date of publication of application: 26.10.2005
(73) Proprietor: Finsbury (Development) Limited, Leatherhead, Surrey KT22 0BA (GB)
(72) Inventor: Tuke, Michael Antony, Guildford Surrey GU1 3TF (GB); Wozencroft, Robert Michael, Epsom Surrey KT19 8SS (GB); Scott, Gareth, London E1 1BB (GB)
(74) Representative: Smaggasgale, Gillian Helen

(56) References cited:
- EP-A- 1 477 120
- WO-A-03/043504
- WO-A-03/055400
- US-A- 6 156 069
- US-A1- 2002 072 805

## Description

The present invention relates to a tool for use in hip resurfacing operations. More particularly, it relates to a head alignment guide.

The efficient functioning of the hip joints is extremely important to the well being and mobility of the human body. Each hip joint is comprised by the upper portion of the upper leg bone (femur) which terminates in an offset bony neck surmounted by a ball-headed portion which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component of the prosthesis can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modern prosthesis, the acetabular component may be coated on its external surface with a bone growth promoting substance which will assist the bone to grow and thereby assist the holding of the acetabular component in place. The bone femoral head will be removed and the femur hollowed using reamers and rasps to accept the prosthesis. The stem portion will then be inserted into the femur.

In some cases, a femoral component of the kind described above may be replaced with components for use in femoral head resurfacing or for use in thrust plate technology.

Although the prosthesis being inserted when the head is being replaced or resurfaced or in thrust plate arrangements is relatively small, the requirement for the surgeon to obtain the necessary access to the hip joint means that it is necessary to make a large incision on one side of the hip. In one technique, a straight incision is made through the skin on the posterior edge of the greater trochanter. In some techniques this incision may be made when the hip is flexed to 45°. By known techniques, the muscles and tendons are parted and held by various retractors such that they do not interfere with the surgeon's access to the hip joint. The hip is then dislocated to provide access to the head of the femur.

It will be acknowledged that it is essential that the replacement surface for the head of the femur should be precisely located in both angular and translation positions of the axis of the femoral neck of the implant. To assist this, in some techniques, the surgeon inserts a pin in the lateral femur. The desired position of the pin will be known from pre-operative analysis of the x-rays. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include those known as the McMinn Alignment Guide available from Midland Medical Technologies Ltd.

Prior art alignment guides of the kind described above generally comprise a hook or aperture which is placed over the alignment pin thus providing a good angular position for the axis of the implant in valgus, varus and ante-version of the neck. The guide will then be adjusted so that a cannulated rod is located such that the aperture therein is directed down the mid-lateral axis of the femoral neck. A stylus having been set to the desired femoral component size is positioned such that it can be passed around the femoral neck. When the stylus can be passed around the femoral neck, the cannulated rod is locked in position. Once the guide is stabilised in this way fine adjustments can be made until the surgeon is happy that the guide is in the required position.

A guide wire can then be inserted though the cannulated rod. This guide wire is then used in the further surgery in which the femoral head is shaped to accept the prosthesis. It will be understood that the alignment guide is an essential tool in the surgical procedure to ensure that the aperture drilled in the femoral head is both central to the femoral neck and at the correct angle of alignment to the femoral neck and that the shaping of the femoral head is accurate for the chosen head size.

Failure to position the alignment guide correctly may have the disastrous effect of allowing the machining of the cylinder of the head during the shaping procedure to "notch" into the neck of the femur. This will predispose the bone to early failure on load bearing.

Other guides are known which are, in use, located on the femoral neck itself. These are used in a similar manner to those described above and may involve some adjustment by the surgeon before he selects the best position.

Whilst the prior art alignment guides are particularly suitable for their function and have reached a high level of acceptance amongst surgeons, there is now a move towards a less invasive surgery in which the required incision is as small as possible and the amount of interaction with healthy tissue is minimised. It is therefore desirable to consider carrying out femoral head replacement or resurfacing without the need to insert the alignment pin so that all of the surgical procedure takes place at the femoral head. Similarly it is desirable to be able to apply thrust plate prosthesis without the need to pre-insert the alignment guide. There is therefore a requirement for an alignment guide which can function without interaction with an alignment pin. In addition, it is desirable that the overall function and safety of the alignment guide be improved. It is further desirable that the alignment guide facilitates the accuracy and ease of use of the instruments that work from the neck.

US 2005/0080426 discloses an apparatus for fitting a protecting femoral neck device. The apparatus includes a clamping base. A fixation element the circumference size can be adjusted according to the size of the femoral neck is mounted on an end of the clamping base and a locating element is mounted on the other end of the said clamping base. An element for shaping the bone can be fixed on the said located element.

US 6156069 discloses at Figure 9 a clamp provided with jaws which grip the neck of the femur between the head of the femur and the main portion of the femoral bone. The two jaws of the clamp are pivoted to the support member so that when the handle is rotated and the canning surface is moved upwardly, the clamp jaws forcibly engage the neck of the femur. A Steiman pin may be advanced through openings in the handle, the threaded actuator and the canning member and enter the head of the femur, hopefully centrally and axially lined with the head and the neck of the femur.

EP 1477120 which was not published until after the priority date of the present application describes a jig for identifying a point on the femoral head which is in alignment with a central axis of the femoral neck. The jig comprises a first member which defines a plane and has means for at least partially receiving the femoral neck. The jig also comprises a guide member which is melted apart from the first member and which defines an axis of right angles to the plane defined by a first member. The axis of the guide member intersects the plane at a point which is a predetermined distance away from the means receiving the femoral neck. In addition, the jig comprises an elongate alignment means which is mounted with respect to the first member and which is spaced apart from, but extends parallel to, the axis of the guide member. There is also described a kit for use in the resurfacing of the femoral head. The kit comprises at least one jig.

Thus according to the present invention there is provided an alignment guide for use in femoral head surgery comprising:
a support arm having a proximal and a distal end;
a ring located at the distal end of the support arm and angled to the plane extending
along the support arm;
a collar located at the proximal end of the support arm; and
a cannulated rod; said rod being a sliding fit in the collar and having teeth located
at the proximal end thereof;
the support arm being configured such that the axis of the bore of the collar passes through the centre point of the ring.

The ring will generally be angled at between 80° and 100° to the plane of the support arm and more preferably will be substantially perpendicular to the support arm.

The ring may be positioned such that the bore of the collar is in line with the centre point of the ring. Alternatively, the centre point may be offset from the axis of the bore. In one arrangement, the alignment guide may include means to enable the user to adjust the position of the ring relative to the axis of the bore.

Locking means may be provided to lock the cannulated rod in a selected position. The locking means may be a locking screw.

The alignment guide is configured such that in use the ring may be located beneath the head of the femur at the neck-head junction. Generally the guide will be configured such that when in position the ring replicates the rim of a resurfacing head prosthesis thereby giving the surgeon a visual indication of the position of the prosthesis prior to commencing the reshaping and drilling of the femoral head. In addition, the ring replicates the inside of the bore of a cylindrical cutting tool which will be used to reshape the femoral head.

In one arrangement, the ring may be sized such that the outside edge of the ring replicates the size of the femoral head. In use, this will enable the surgeon to simply hold the ring against the femoral head to visually assist whether the correct ring size is being used for a particular patient. In this arrangement, the ring may include markings which when the ring is used as a size check, can be positioned at the desired position for the skirt of the prosthesis.

In femoral head resurfacing techniques, the surgeon will shape the head of the femur to fit within the cavity of a resurfacing prosthesis. This generally involves a number of shaping steps including the removal of the dome of the femoral head by means of a saw. It is important that the saw cut is made in the correct position and plane so that an accurate fit with the prosthesis can be achieved. Since the ring replicates the position of the rim of the resurfacing head prosthesis, a measure can be taken from the ring to the position and plane of the required cut. In one arrangement, to facilitate correct cutting, the alignment guide of the present invention may also include a saw cutting guide located on the support arm at an appropriate distance from the ring to assist the surgeon to cut in the correct position. The saw cutting guide may be movable on the support arm such that the surgeon can select the required position or, in one alternative arrangement, may be preset for a particular size of resurfacing prosthesis. Where the saw cutting guide is movable, a locking means may be provided to lock the cutting guide in the required position.

It is also possible for the position of the cut to remove the dome of the femoral head to be calculated from the top of the dome. Thus in an alternative arrangement a saw cutting guide may be located on the collar, or where present on the cannulated rod, or on another element of the guide such that when the rod is in position, the guide will illustrate the correct position for the cut. In this arrangement separate guides may be provided for each head size of resurfacing head prosthesis.

The ring may be of any suitable configuration. In one arrangement, the ring is a C-ring so that in use the guide can be placed in position by passing the neck of the femoral head through the open space in the ring. It will be understood that the size and position of the break in the C-ring may mean that the ring may be placed around the femoral neck in one orientation and then the device rotated to the desired position for surgery.

In another arrangement the ring may have a portion which is hinged to the remainder of the ring and which in an open orientation will enable the guide to be placed in position around the neck of the femoral head. The hinged portion can then be closed to complete the ring. Thus the hinged portion may be regarded as a gate or door.

In a still further arrangement the ring may be formed of two arms which are each hinged to allow movement to an open position such that the guide may be located in position. When in the closed position in use, the two arms will close around the neck of the femur. In one arrangement, the two arms will be sized such that when in the closed position, they complete a circle but they may simply form a segment of a circle. The two arms may be of the same or different lengths. Thus where the arms, when in the closed position, complete a circle, the two arms may meet at a point opposite the point of connection to the support arm or may be at another point on the circumference of the circle. In one alternative arrangement one arm may be fixed and the other may be adjustable thereto.

In a further alternative arrangement, the ring may be adjustable. For example, an iris which will expand to enable the ring to be passed over the femoral head and then contracted to fit around the neck of the femur.

In one arrangement, the different alignment guides may be provided each with a different size ring permanently attached thereto. However, in one alternative arrangement, the ring component may be demountable so that different size rings may be used as dictated by the size of the patient's femur. According to a second aspect of the present invention there is provided a kit comprising the alignment guide of the above first aspect and a plurality of rings.

Whichever arrangement is used, it will be understood that when in position, the ring will generally replicate the rim of a particular size of resurfacing head prosthesis.

Where separate rings are provided, they may be provided with a peg extending upwardly from the ring which engages with the support arm in any suitable manner. In one arrangement, the peg may be shaped to be a sliding fit in a channel in the support arm. In an alternative arrangement, the peg may be of tubular cross-section and may when connected to the support arm extend around the support arm. The peg may be fastened to the support arm by any suitable means. In one arrangement, a locking means, such as a screw lock, may be provided. Where a saw cutting guide is provided which includes a locking means, the means for locating the locking means and for holding the ring in place may be the same. In one arrangement, the peg of each interchangeable ring will be provided with an aperture into which the saw guide can be locked. In this arrangement, the aperture on each ring will be located in the correct position to ensure that the correct positioning of the saw guide for a particular ring size is ensured.

In one arrangement, the guide may be provided with a plurality of rings each sized to correspond to a respective replacement femoral head size.

In one arrangement the selection of a guide with an appropriate sized ring component or the appropriate ring where the ring is demountable will determine, or will assist the surgeon to determine, the correct head size for the patient on which the operation is being carried out.

It will be understood that the ring component is preferably chosen to fit onto the head-neck junction of the femur once bony protuberances such as osteophytes have been removed.

It will be acknowledged that the ring, whether a full or part circle such as the C-ring, does not have to be shaped as at least part of a circle. For example, the ring may be at least part of the portion of another shape such as a triangle, square, pentagon, hexagon, heptagon, octagon or other multi-sided shapes. It may be of a shape similar to that of a horse-shoe. In an alternative arrangement it may be configured to the shape of the femoral neck or the head/neck junction. Thus it should be understood that any reference to "circle" and "ring" should be construed to include these other shapes. "Circle" and "ring" are simply used for ease of reference.

Where the cannulated rod is present it may be in a fixed position with respect to the collar or it may be adjustable thereto. The collar itself may be adjustable with respect to the support arm. Once in the required position the collar, and where present the rod, will preferably be lockable such that once locked further movement is prevented. The locking means may be of any suitable arrangement. In one arrangement, a locking screw may be used.

The cannulated rod, or where the rod is absent, the collar, will in use, enable the surgeon to position a guide wire or a drill onto the head of the femur or where an aperture has already been formed in the head, into the head of the femur. In one arrangement the collar and/or the cannulated rod where present may have a slot extending along at least a part of the length of the rod to assist in removing the collar and/or rod from the guide wire once it is in position. Where present a slot extending along at least a part of the length of the collar may assist in removing the whole guide or just the rod from the guide wire or drill once it is in position.

The teeth located at the proximal end of the cannulated rod will, in use, grip into the surface of the femoral head. These teeth help to clamp the alignment guide in position and to stabilise the alignment guide by tensioning the rod against the ring and locking the rod.

The cannulated rod may additionally function as a measuring or gauging device and to assist this the surface of the rod may include measuring indica. Additionally or alternatively, the guide wire may include a measuring or gauging device.

A flag and/or guide pin may be included at the distal end of the support arm and where present will generally extend below the position of the ring. The flag and/or guide pin may be integral with the support arm or may be provided as a separate fitting. This flag or pin will serve as a visual guide to assist the surgeon to check that the alignment guide is in the correct alignment with the medial side of the femoral neck to ensure the correct valgus-varus alignment and the correct ante-version angles. A second such arrangement of flag and/or pin may be provided to assist in aligning the alignment guide with the ante-version of the femoral neck.

The alignment guide of the present invention may additionally include stylus means of the kind known in the prior art.

At least a portion of the support arm may be shaped to provide a comfortable position for the user's fingers in use. One or more additional handles of any suitable configuration may be used.

The alignment guide of the present invention may be used in combination with an elongate distal alignment guide which is described in more detail below.

The alignment guide of the present invention may be used in a method for preparing the head of a femur for femoral head resurfacing wherein the method comprises:
exposing the head of a femur;
locating the alignment guide according to the above first aspect beneath the head of the femur at the head-neck junction; and
machining the head of the femur.

During the surgery, the top of the head of the femur may be cut on the alignment guide either after or before placing the guide wire or drilling a larger hole via the collar or rod. This larger hole may be the definite hole diameter required of approximately 8 mm and drilled to an appropriate depth for the implant to be used. A check may be made with a stylus once the alignment guide is removed and cylinder cutters used guided over a peg placed in the hole. These cutters are arranged such that the diameter cut will be correct for the head size chosen and will bottom on the top of the cut head such that the teeth of the cutter do not dangerously over-sail the head-neck junction and cause soft tissue damage or neck notching.

Thus the method preferably comprises:
exposing the head of the femur;
locating the alignment guide according to the above first aspect beneath the head of the femur at the head-neck junction;
inserting a drill through the collar and drilling a well into the head of the femur;
removing the drill;
removing the top of the head of the femur;
removing the alignment guide;
inserting a guide rod into the well;
locating a sleeve cutter on the guide rod and cutting the head; and
optionally chamfer cutting the head.

The correct axis for insertion of the guide wire into the head of the femur is approximately 30 degrees from the sagittal plane axis of the femur and in anteversion to allow for the natural offset in each position. Thus the alignment guide of the present invention is configured such that in use the cannulated bore will be located such that the guide wire or drill is inserted at the correct angle. The arrangement of the present invention allows the surgeon to place, and to visually check that the alignment guide is in the correct orientation, and position centered on the femoral head-neck junction.

It will be understood that whilst the alignment guide of the present invention offers particular advantages for minimal invasive surgery, it can also be used in conventional surgical techniques.

The present invention will now be described by way of example with reference to the accompanying figures in which:
- Figure 1: is a side view of the alignment guide of one arrangement of the present invention;
- Figure 2: is a perspective view from behind of the alignment guide Figure 1;
- Figure 3: is a side view of the alignment guide of Figure 1 in use;
- Figure 4: is a perspective view from the side of a second arrangement of the present invention;
- Figure 5: is a side view of the alignment guide of Figure 4 in use;
- Figure 6: is a perspective view from the side of a third arrangement of the present invention;
- Figure 7: is a perspective view of the alignment guide of Figure 6 including one arrangement of a saw cutting guide;
- Figure 8: is a perspective view of the alignment guide of Figure 6 including an alternative arrangement of a saw cutting guide;
- Figure 9: is a partially cutaway schematic view of the alignment guide of Figure 8;
- Figure 10: is a partial cross-section of the alignment guide of Figure 8 taken through the plane extending through the aperture in the C-ring;
- Figure 11: is an exploded view of the ring of Figure 8;
- Figure 12: is an illustration of the alignment guide of Figure 8 partially cut-away and showing the ring in the open position from the front;
- Figure 13: is a perspective view of the illustration of Figure 12;
- Figure 14: is an illustration of the alignment guide of Figure 8 partially cut-away and showing the ring in the closed position from the front;
- Figure 15: is a stepwise illustration of the alignment guide of present invention in use;
- Figure 16: is a schematic composite illustration to show the relationship between the features of the prosthesis and the features of the alignment guide;
- Figure 17: is a side view of a fourth arrangement of the present invention in use;
- Figure 18: is a perspective view of the alignment guide of Figure 17;
- Figure 19: is a perspective view of the alignment guide of Figure 17 including one arrangement of a saw cutting guide;
- Figure 20: is a perspective view of the alignment guide of Figure 17 including an alternative arrangement of a saw cutting guide;
- Figure 21: is a partially cutaway schematic view of the alignment guide of Figure 20;
- Figure 22: is the alignment guide of Figure 17 including a guide wire support and guide wire;
- Figure 23: is a close up of the guide wire support and guide wire of Figure 22;
- Figure 24: is a side view of the alignment guide of Figure 22 illustrating the locking mechanism;
- Figure 25: is a detailed partially cut away detailed view of portion A of Figure
- Figure 26: is a partially cut away schematic view of the alignment guide of Figure 17 with showing the ring in the closed position;
- Figure 27: is a perspective view from above of the alignment guide of Figure 26 with the ring in the closed position;
- Figure 28: is an enlarged detail of section X of the alignment guide of Figure 26 (scale 1: 1);
- Figure 29: is an enlarged detail of section V of the alignment guide of Figure 26 (scale 2:1);
- Figure 30: is a partially cutaway detailed schematic view of the alignment guide of Figure 17 with the ring in the open position;
- Figure 31: is a perspective view from above of the alignment guide of Figure 30 with the ring in the open position;
- Figure 32: is an enlarged detail of section X of the alignment guide of Figure 30 (scale 1:1);
- Figure 33: is an enlarged detail of section V of the alignment guide of Figure 30 (scale 2:1);
- Figure 34: is a stepwise illustration of the use of the alignment guide of Figure 17;
- Figure 35: is a schematic illustration of the ring portion of the alignment guide of Figure 17 illustrating the alignment of the components of the ring component with a replacement femoral head;
- Figure 36: is a schematic view of an alternative use of the ring portion of the alignment guide of Figure 17 to verify selection of the correct size of replacement femoral head;
- Figure 37: is a stepwise illustration of an alternative use of the alignment guide of Figure 17;
- Figure 38: is a schematic illustration of the alignment guide of the present invention in combination with a distal alignment guide showing alignment with the femur;
- Figure 39: is a schematic illustration of the alignment guide of the present invention in use in combination with the distal alignment guide located against the knee;
- Figure 40: is a perspective view of the arrangement of Figure 38; and
- Figure 41: is an enlarged detail of V of Figure 40.

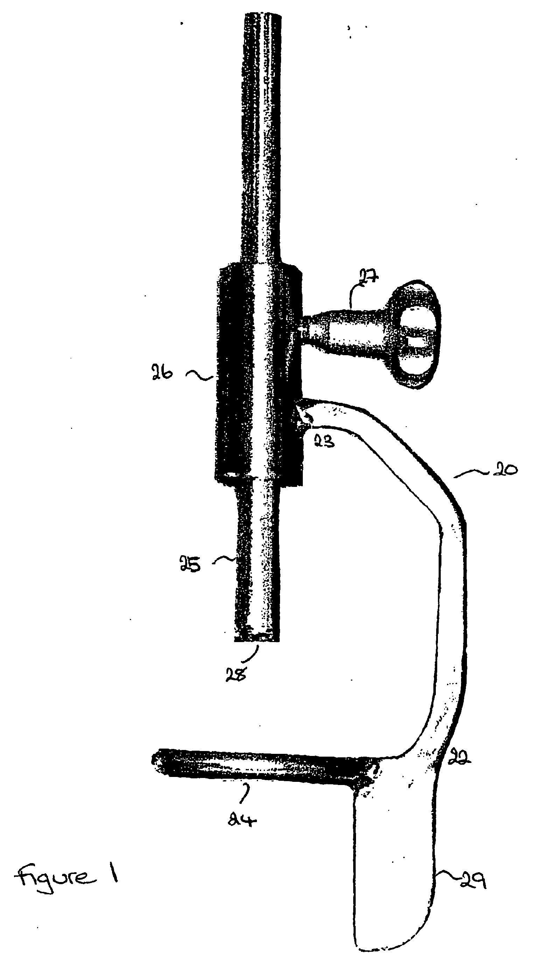

As illustrated in Figure 1, the alignment guide 20 of one embodiment of the present invention comprises a support arm having a distal 22 and a proximal 23 end. A ring 24 is located at the distal end of the arm and extends therefrom such that it is at right angles to the plane of the arm. However, it will be noted that the arm is shaped to allow the arm, in use, to fit around the femoral head. A cannulated rod 25 is a sliding fit in a collar 26 located at the proximal end of the support arm. The rod 25 is situated such that the cannula is directly above the centre of the ring. A locking screw 27 is provided to enable the rod 25 to be clamped in the desired position. Teeth 28 are provided on the end of the rod which in use will come into contact with the femoral head.

A flag 29 may be present to assist the surgeon to visually confirm that the alignment guide is in the desired position.

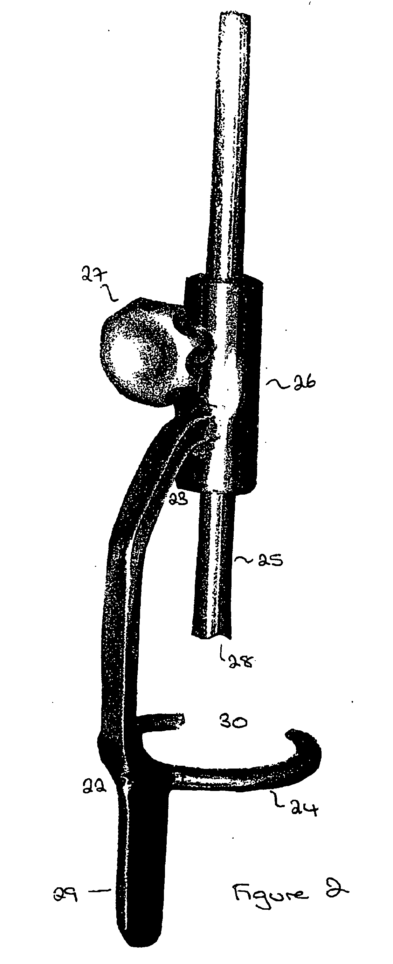

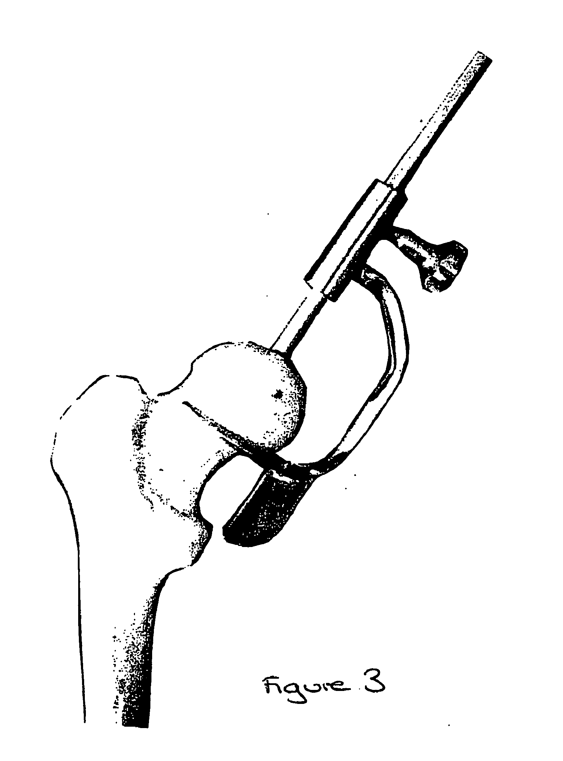

The alignment of the various components can be seen in Figure 2. In the illustrated arrangement, the ring 24 may be a C-ring which has an aperture 30. In use this aperture will enable the ring to be passed around the neck of the femur as illustrated in Figure 3. The surgeon will then manipulate the alignment guide to the desired position and then lower the rod 25 such that the teeth 28 come into contact with the surface of the femoral head. The screw lock will then be operated to lock the position of the rod.

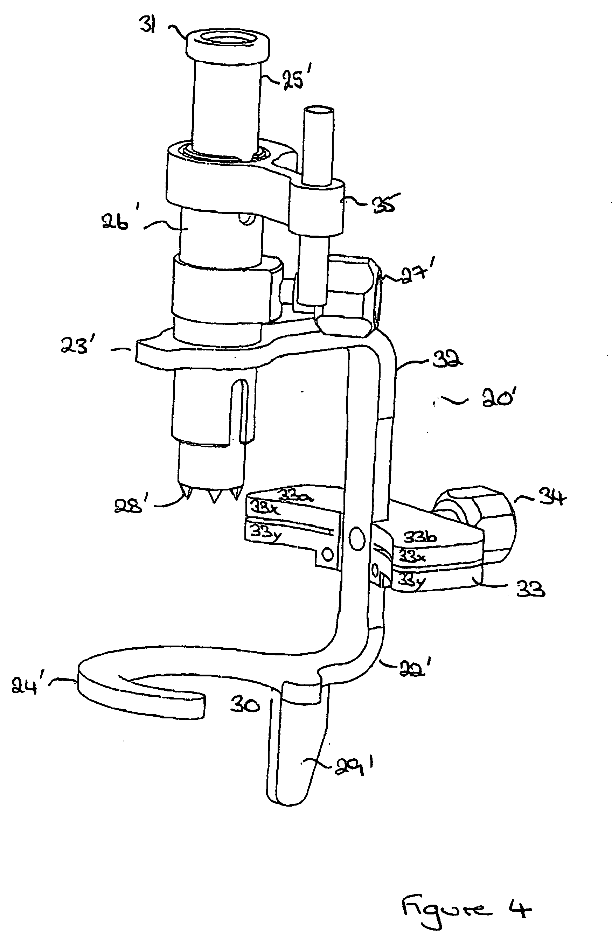

An alternative arrangement is illustrated in Figure 4. Here the alignment guide 20' comprises a support arm 32 having a distal 22' and a proximal 23' end. A ring 24' is located at the distal end of the arm and extends therefrom such that it is at right angles to the general plane of the arm. However, it will be noted that the arm is shaped to allow the arm to fit around the femoral head in use. A cannulated rod 25' is a sliding fit in a collar 26' located at the proximal end of the support arm. The rod 25' is situated such that the cannula is located directly above the centre of the ring. The rod includes a rim 31 to prevent the rod passing completely through the collar in a downward direction. Thus the rim acts as a stop. A locking screw 27' is provided to enable the rod 25' to be clamped in the desired position. Teeth 28' are provided at the end of the rod which in use will come into contact with the femoral head.

A flag 29' may be present to assist the surgeon to visually confirm that the alignment guide is in the desired position.

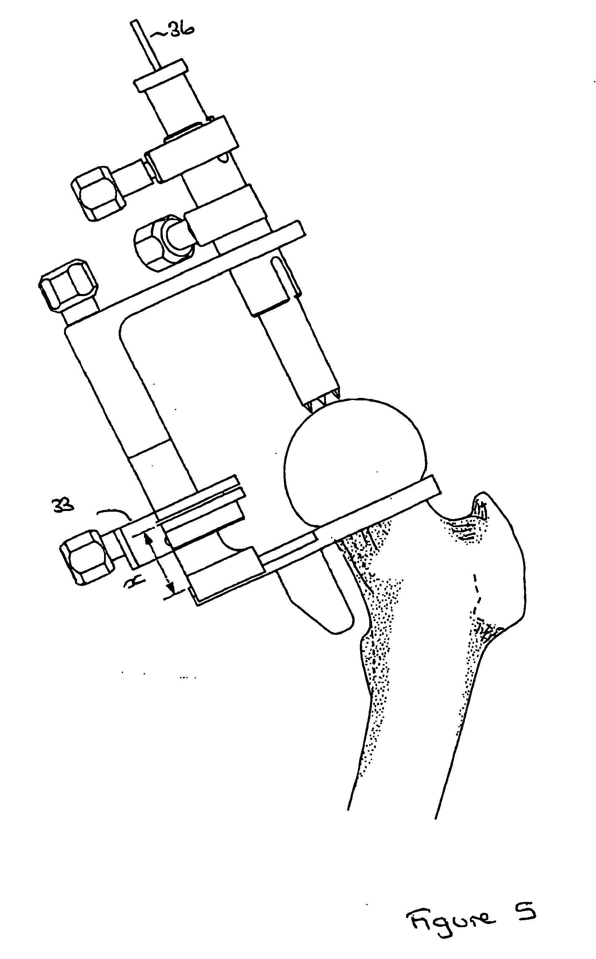

In the illustrated arrangement, the ring 24' is a C-ring which has an aperture 30' set at one side of the alignment guide. Thus one arm of the C is substantially longer than the other. In use this aperture will enable the ring to be passed around the neck of the femur as illustrated in Figure 5. The surgeon will then manipulate the alignment guide to the desired position and then lower the rod 25' such that the teeth 28' come into contact with the surface of the femoral head. The screw lock will then be operated to lock the position of the rod.

The illustrated embodiment of Figure 4 also includes one type of suitable saw cutting guide 33. It will be understood that a saw cutting guide may also be provided in the embodiment of Figure 1. The illustrated saw cutting guide is slidably connected to the support arm 32 and includes a lockable saw guide screw 34 to enable the saw cutting guide to be locked in the correct position. The saw cutting guide comprises two arms 33a and 33b and each arm includes two wings 33x and 33y surrounding a slot through which in use the saw blade will be placed. The configuration of the saw cutting guide assists the surgeon to keep the blade in the desired plane.

As illustrated in Figure 5, the saw cutting guide will be located at a position a distance x from the ring of the alignment guide of the present invention such that the cut in the head of the femur is made at the optimum position.

In the illustrated arrangement and as shown in Figure 4, a guide wire support 35 may be provided to enable a guide wire to be used to assist the surgeon to align the alignment guide correctly. Again this support may be included in the arrangement of Figure 1.

As illustrated in Figure 5, in use a guide wire 36 may be present.

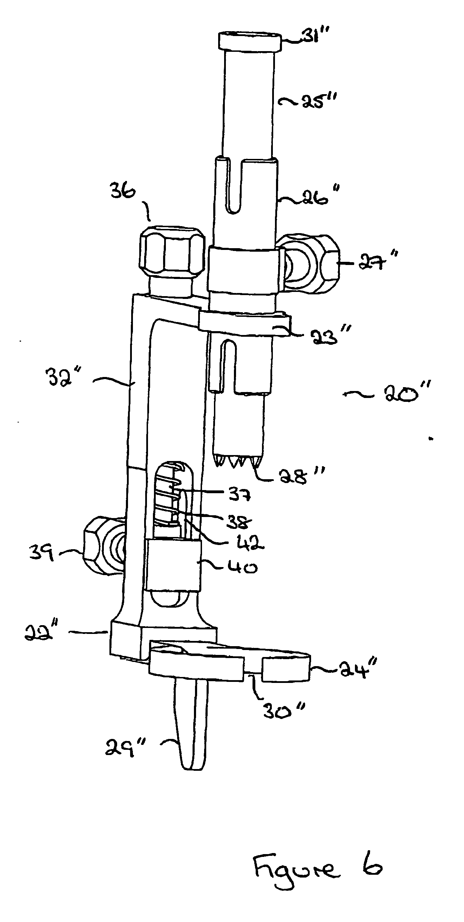

A still further alternative arrangement is illustrated in Figure 6. Here the alignment guide 20" comprises a support arm 32" having a distal 22" and a proximal 23" end. A ring 24" is located at the distal end of the arm and extends therefrom such that it is at right angles to the general plane of the arm. In this arrangement, the arm is shaped to allow the arm, in use, to fit around the femoral head. A cannulated rod 25" is a sliding fit in a collar 26" located at the proximal end of the support arm. The rod 25" is situated such that the cannula is located directly above the centre of the ring. The rod includes a rim 31" to prevent the rod passing completely through the collar in a downward direction. Thus the rim acts as a stop. A locking screw 27" is provided to enable the rod 25" to be clamped in the desired position. Teeth 28" are provided at the end of the rod which in use will come into contact with the femoral head.

A flag 29" may be present to assist the surgeon to visually confirm that the alignment guide is in the desired position.

In the illustrated arrangement, the ring 24" is a C-ring which has an aperture 30" located opposite the support arm. In the illustrated arrangement, the aperture is small such that the ring cannot be passed around the neck of the femur. Here, as will be discussed in more detail below, at least one arm of the ring may be movable such that arms move apart and the aperture becomes wide enough to allow it to be passed over the neck of the femur. A knob 36 connects to the ring via a rod 37 and spring 38 which enables the user to open and close the arms of the ring. A brace locking screw 39 and block 40 braces the system. Grooves 42 in the internal sides of the support arm enable the screw and block to be moved.

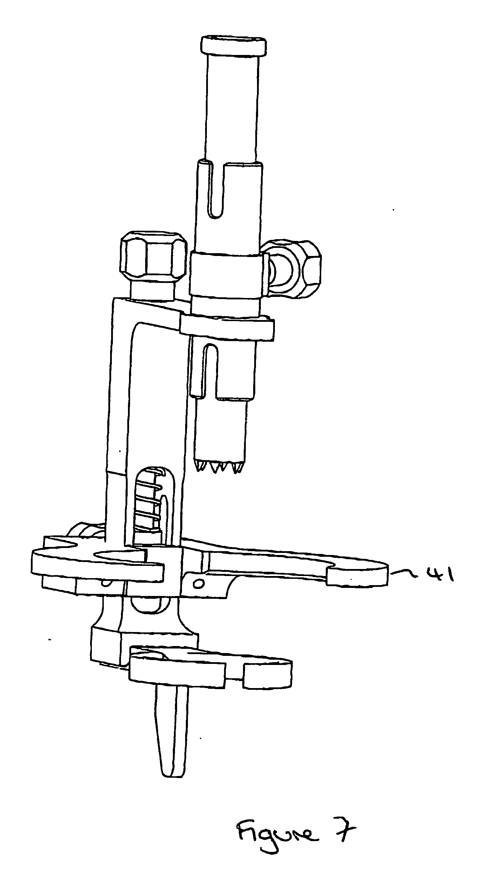

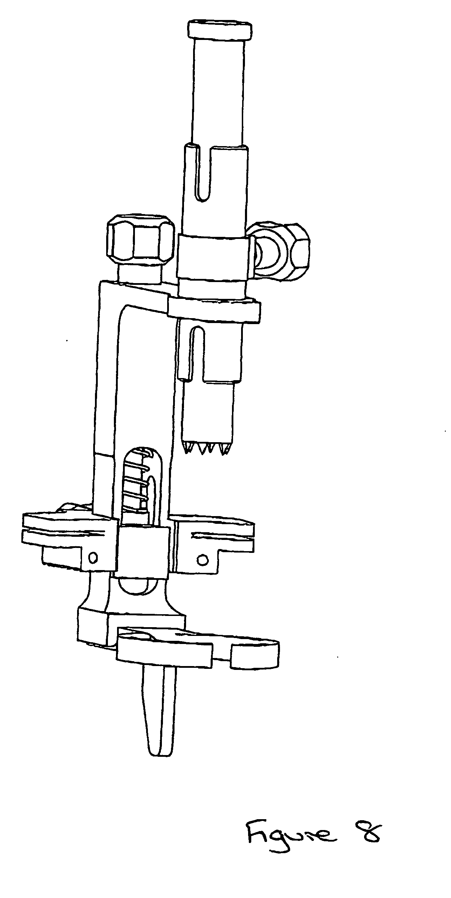

As illustrated in Figure 7, the alignment guide of Figure 6 may also include a saw cutting guide 41. This saw cutting guide is a different configuration of saw cutting guide to that illustrated in Figure 4 and comprises a plate of C-configuration such that the saw can be laid across the arms. It will be understood that the saw cutting guide could be the guide of Figure 4 as illustrated in Figure 8. Similarly the saw cutting guide of Figure 7 could replace that of Figure 4 or could be included with the alignment guide of Figure 1. Whichever saw cutting guide is used, it replaces the block 40 such that the locking screw 39 serves to lock the saw cutting guide in the desired position.

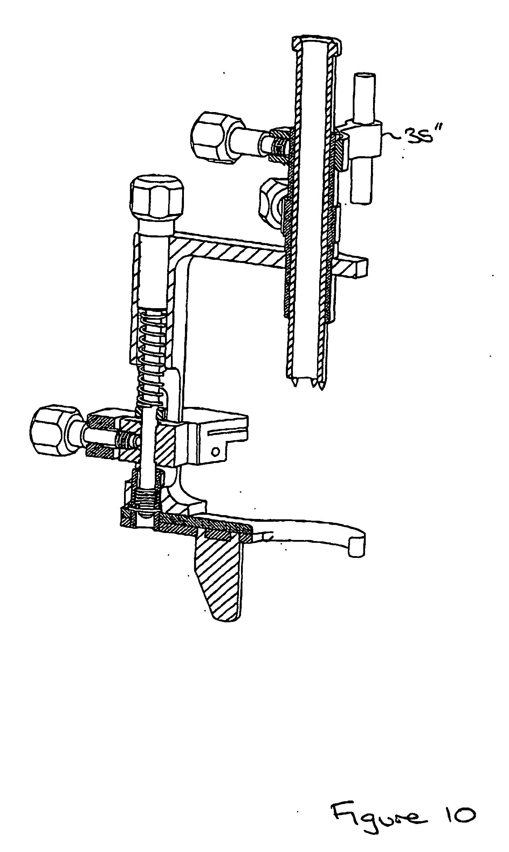

The arrangement of Figure 6 may also include a guide wire support 35" as illustrated in Figure 10.

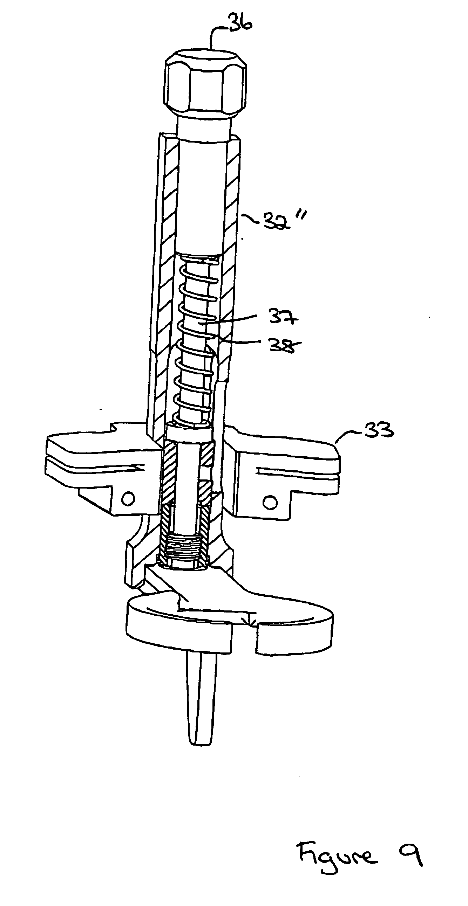

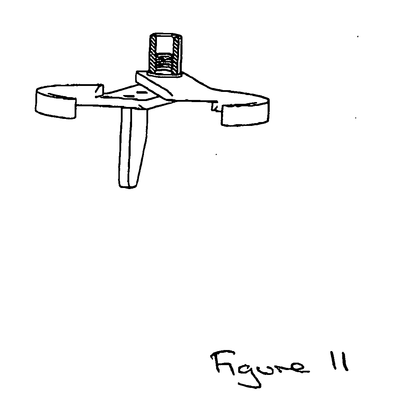

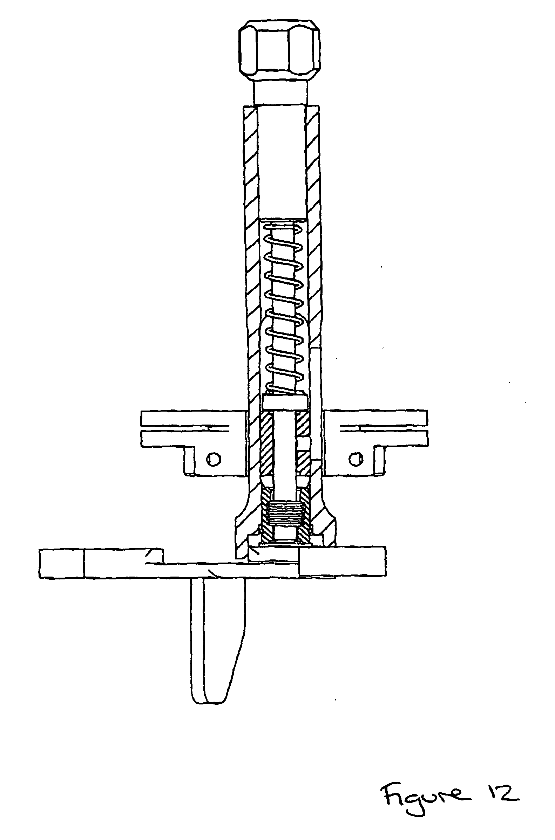

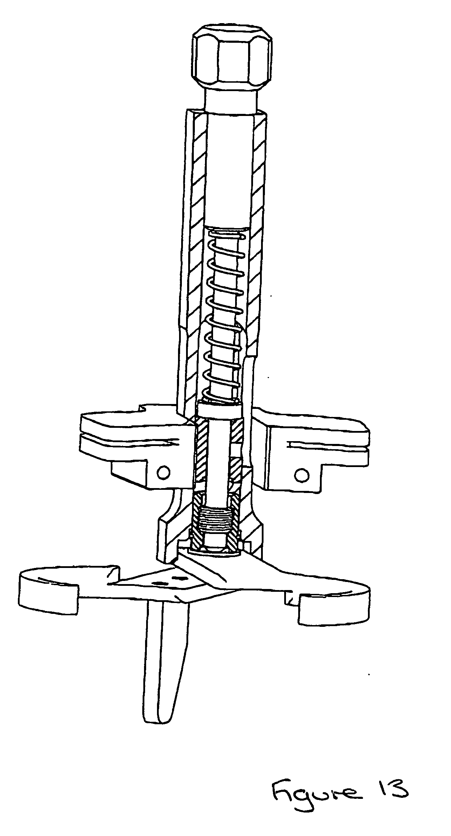

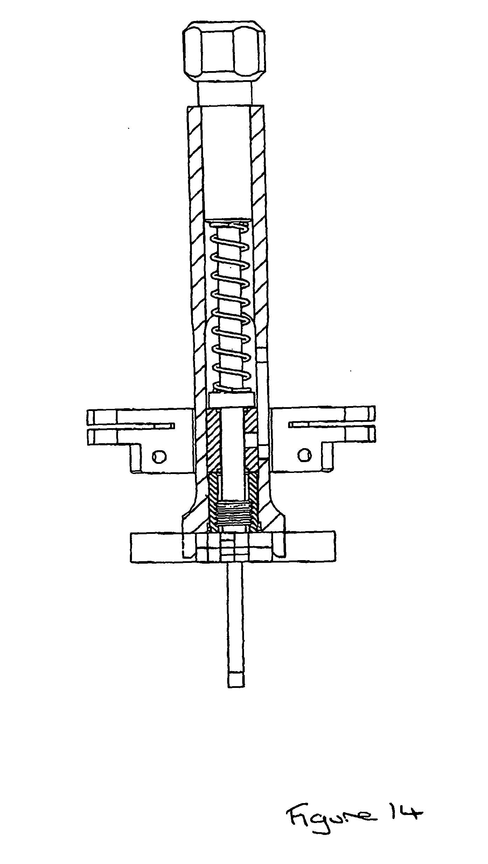

The arrangement within the support arm is illustrated in Figures 9, 10 and 12 to 14. The rod 37 passes through the support arm and has a threaded end which enables it to connect to the C-ring. As shown in Figure 11, a corresponding threaded female member is provided on the C-ring. As the nut 26 is rotated, the rod 37 will rotate such that the arms of the ring can be opened.

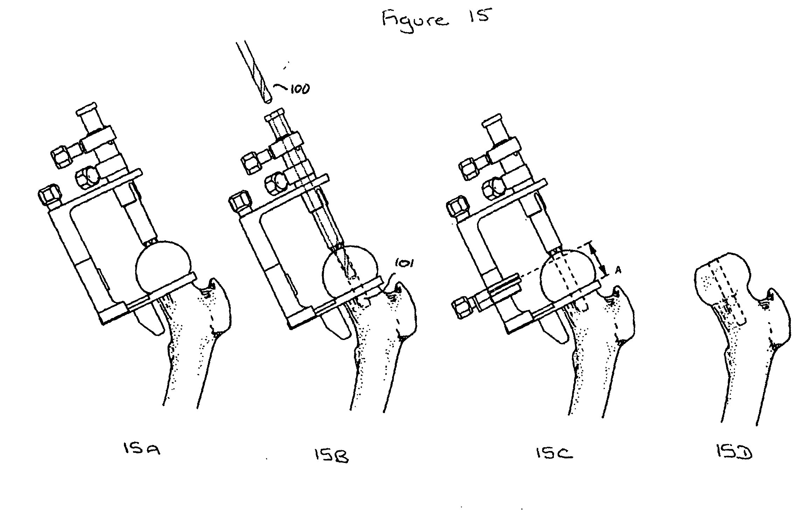

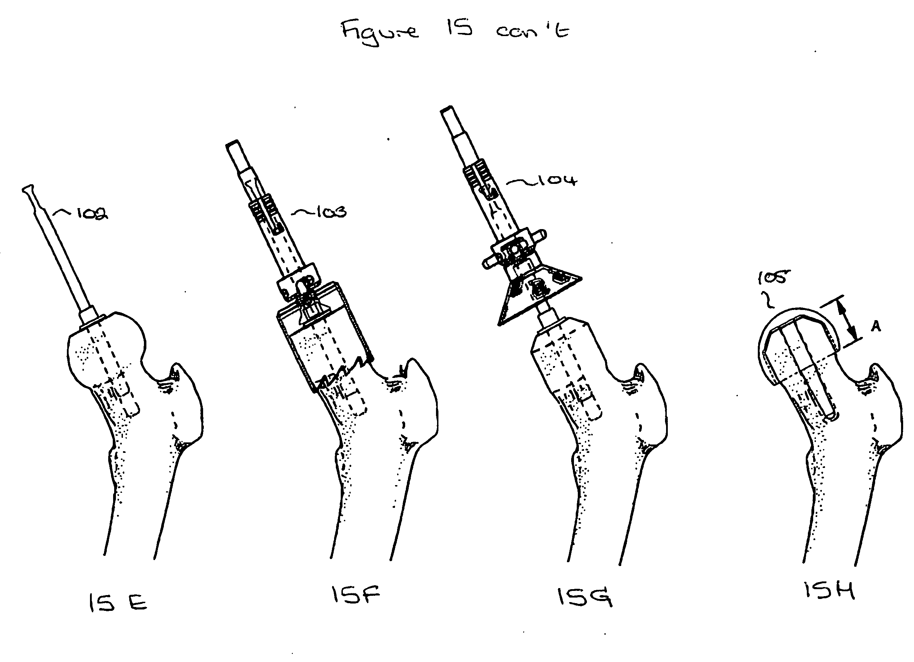

As illustrated in Figure 15, in use the ring of the alignment guide of the present invention is located beneath the head of the femur at the head-neck junction (Figure 15A). A drill 100 is then inserted into the collar, optionally through the bore of the cannulated rod, and a well 101 is drilled in the head of the femur (Figure 15B). The top of the head of the femur is then removed by sawing using the saw guide when present to ensure that the cut is the correct distance A from the ring of the alignment guide which represents the skirt of the prosthesis (Figure 15C). It will be understood that it some arrangements, the top of the femur may be removed before the well is drilled. The alignment guide is then removed (Figure 15D). A guide rod 102 is then inserted into the well (Figure 15E). This guide rod is then used to locate and correctly angle a sleeve cutter 103 and a chamfering tool 104 (Figures 15F and 15G). The femoral head resurfacing prosthesis 105 can then be applied as illustrated in Figure 15H. It can be seen that the distance from the cut head of the femur to the edge of the skirt of the prosthesis is A.

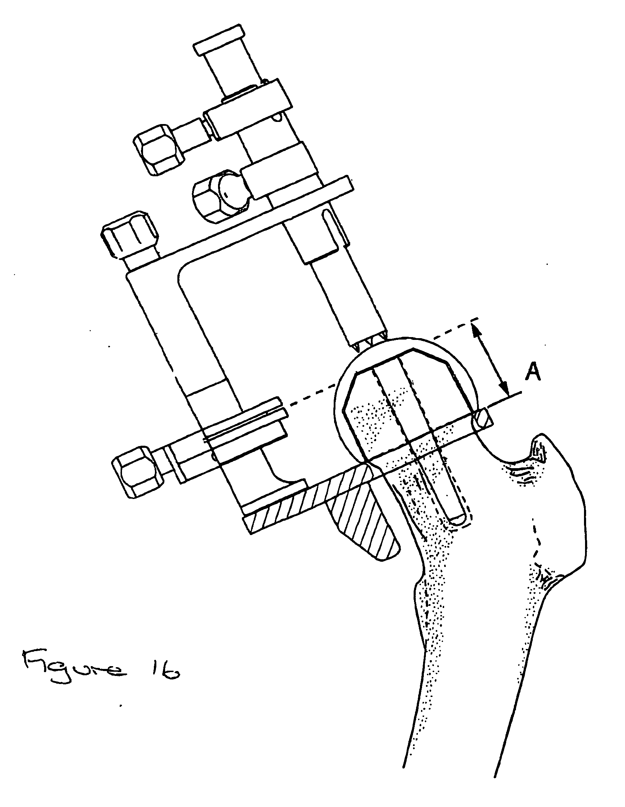

Figure 16 is a schematic representation of the alignment guide of the present invention illustrating the relationship of the ring and the saw guide to the head of the prosthesis.

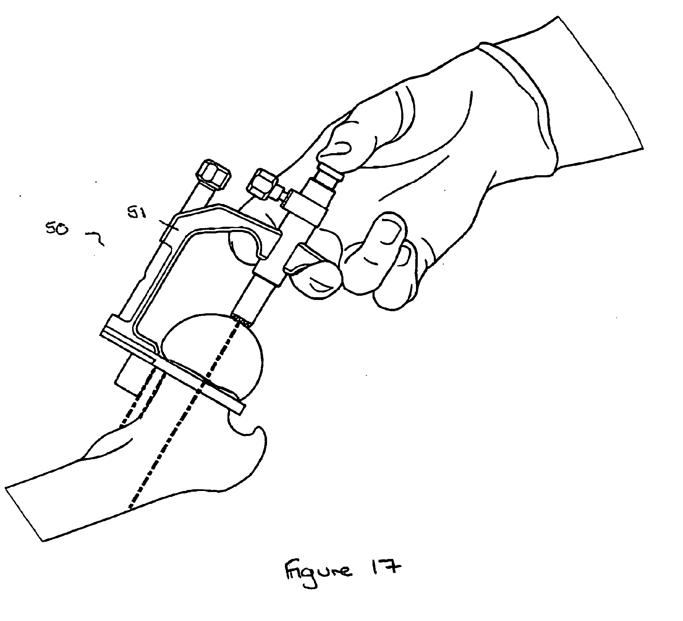

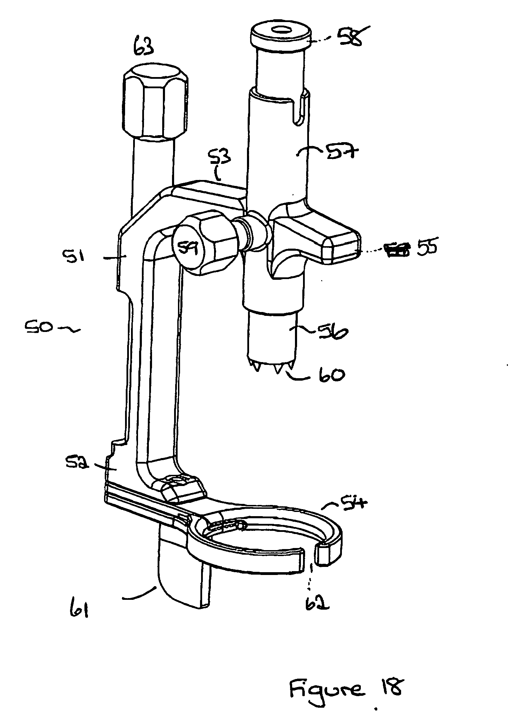

A still further arrangement is illustrated in Figures 17 and 18. Here the alignment guide 50 comprises a support arm 51 having a distal 52 and a proximal 53 end. In this arrangement, the arm is shaped to allow the arm, in use, to fit around the femoral head. The underside of the proximal end of the arm may be shaped to allow the surgeon's finger to sit comfortably against the arm. A cooperating handle member 55 may be provided which is also shaped on the underside to allow the surgeon to handle the alignment guide in a convenient and comfortable manner. It will be understood that the arms of the other arrangements may be similarly shaped and may also be provided with corresponding handle members.

A ring 54 is located at the distal end of the arm and extends therefrom such that it is at right angles to the general place of the arm A cannulated rod 56 is a sliding fit in a collar 57 located at the proximal end of the support arm. The rod 56 is situated such that the cannula is located directly above the centre of the ring. The rod 56 includes a rim 58 to prevent the rod passing completely through the collar in a downward direction. Thus the rim acts as a stop. A locking screw 59 is provided to enable the rod 56 to be clamped in the desired position. Teeth 60 are provided at the end of the rod which in use will come into contact with the femoral head.

A flag 61 may be present to assist the surgeon to visually confirm that the flag is in the desired position. The correct alignment of the flag is shown in Figure 17.

In the illustrated arrangement, the ring 54 is a C-ring which has an aperture 62 located opposite the support arm In the illustrated arrangement, the aperture is small such that the ring cannot be passed around the neck of the femur. Here, as will be discussed in more detail below, at least one arm of the ring may be movable such that arms move apart and the aperture becomes wide enough to allow it to be passed over the neck of the femur. A knob 63 connects to the ring in a manner which will be described in more detail below and which enables the user to open and close the arms of the ring.

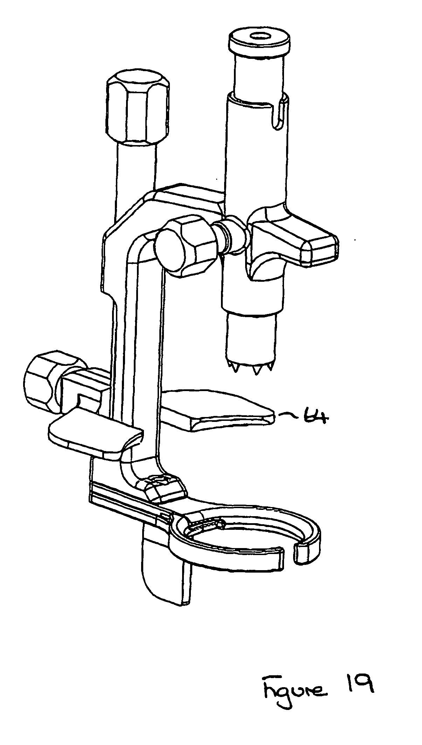

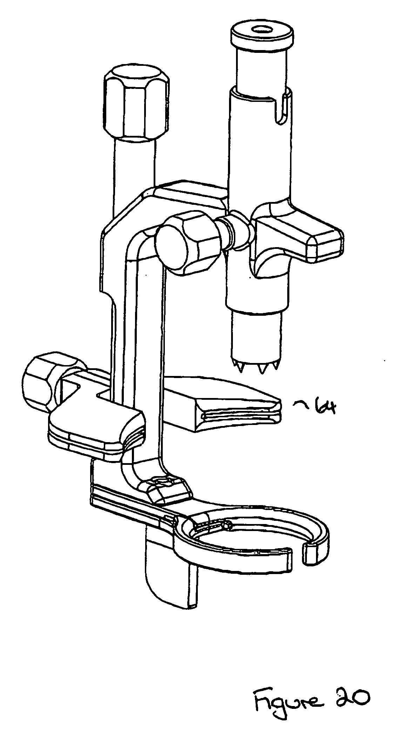

As illustrated in Figure 19, the alignment guide of Figure 17 may also include a saw cutting guide 64. This saw cutting guide is of a similar configuration to that illustrated in Figure 7 and comprises a plate of C-configuration such that the saw can be laid across the arms. As illustrated in Figure 20, the saw cutting guide 64 could be of the type described in detail above in connection with the guide of Figure 4.

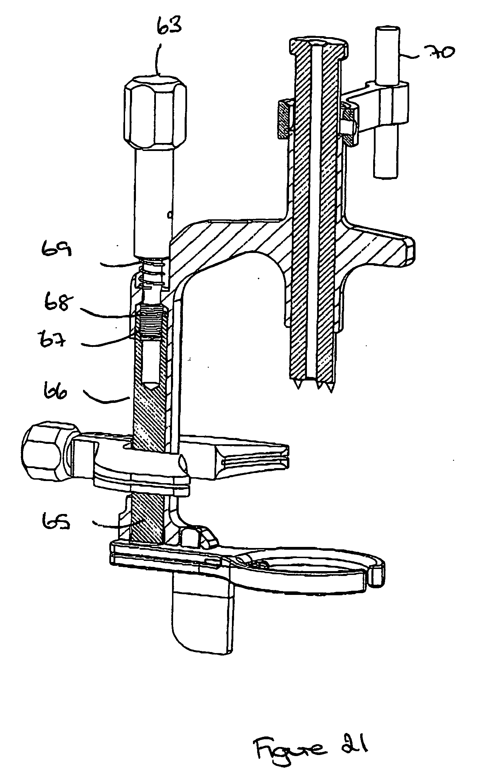

As illustrated in Figure 21, in the present arrangement, the ring is demountable and is provided with a peg 65 which in use extends into a channel 66 in the arm. The upper end of the peg 65 is provided with a female means which is preferably a threaded aperture 67 and which can receive a locking means, which is preferably a screw 68 extending from the knob 63 such that when the screw engages the threaded aperture, the peg and hence the ring is held in place. A spring 69 may be provided to bias the locking mechanism. As will be discussed in more detail below, partial turning of the knob 63 will enable the arms of the ring to move from the open to the closed position.

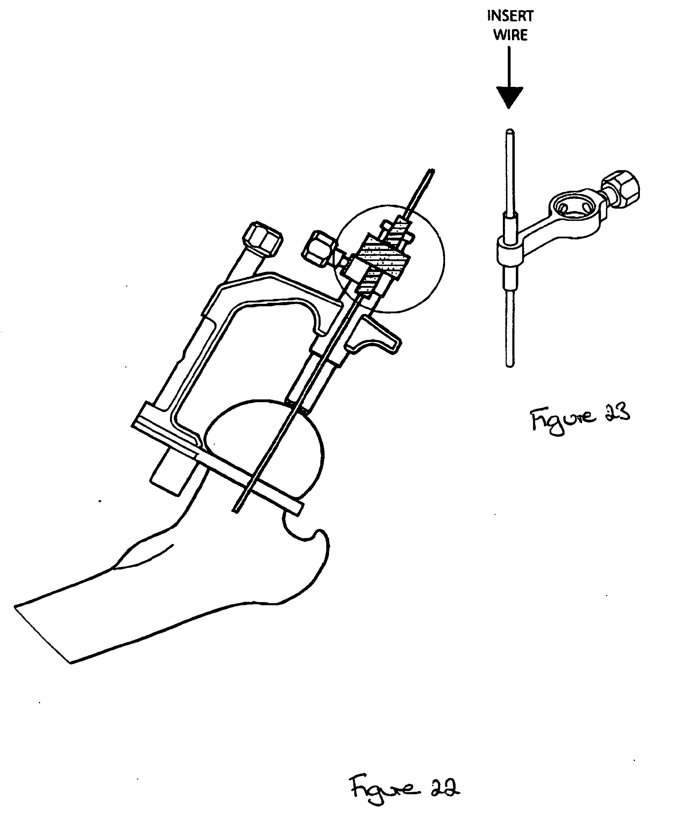

As illustrated in Figures 21, 22 and 23 a guide wire support 70 may be provided to enable a guide wire to be used to assist the surgeon to align the alignment guide correctly. The location of the guide wire support and the guide wire is illustrated in Figure 22 and 23.

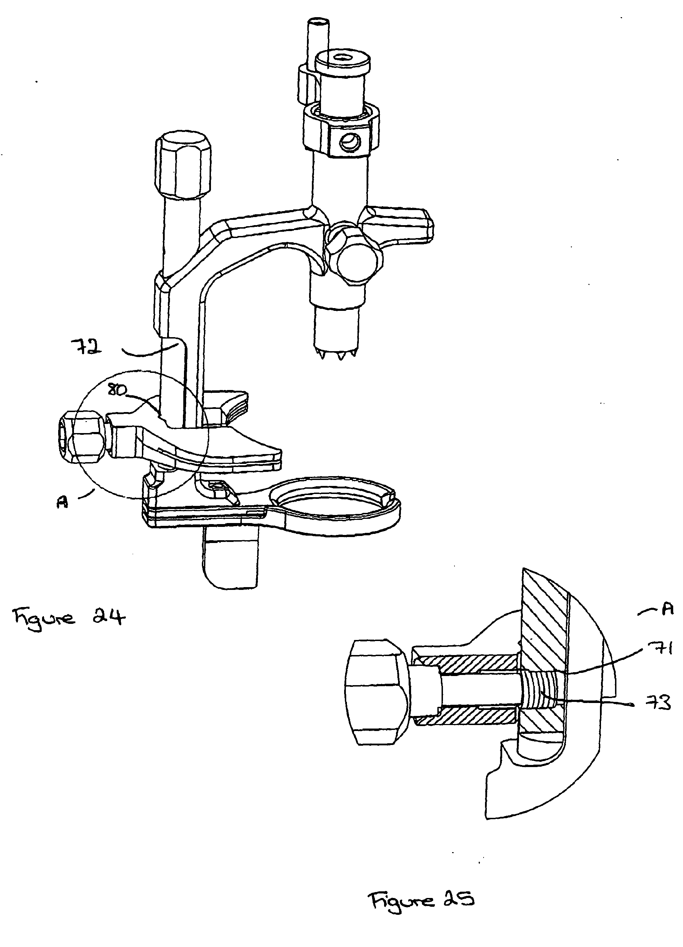

In one arrangement, and in particular where the ring is demountable such that different sized rings may be used with the alignment guide, an aperture 71 (as illustrated in Figure 25) may be provided in the peg 65 extending from the ring. The position of the aperture on the peg will be in a different position for each ring size and will represent the correct positioning for the saw cutting guide for a particular femoral head prosthesis and will correspond to that having the size represented by the ring. In this arrangement, the arm will include an opening 72 (Figure 24) such that at least a portion of the peg which includes the aperture is exposed.

In use, once a ring size has been selected, and the peg has been inserted into the arm and locked in place, the saw guide may be connected to the aperture. Generally the cutting guide will be held in place by a saw cutting guide screw means which has a threaded component which will engage cooperating threads in the aperture. A portion of the cutteing guide which extends around the peg may include a notch 80. The interaction between notch 80 and the peg will serve to ensure that the peg is retained in the correct orientation.

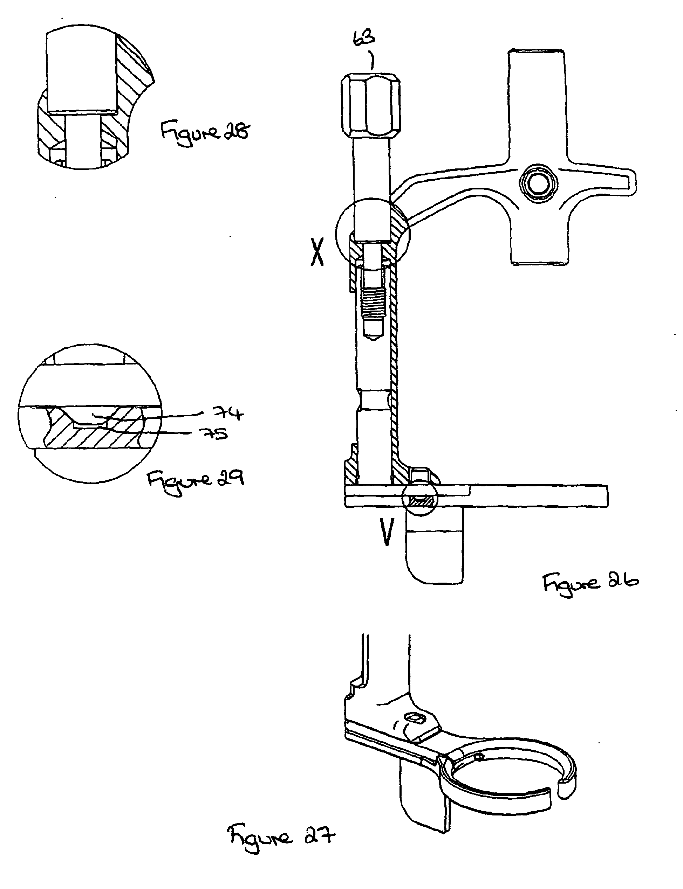

Figures 26 to 33 illustrate the means for opening and closing the arms of the ring in an arrangement in which the two arms of the ring are formed from respective components which are located one below the other at the point of contact. In this arrangement, the lower arm will generally include a cut away portion on the upper side thereof at the point of engagement with the upper arm which will interlock with a corresponding cut away portion on the underside. This arrangement will allow the arms to lie one above the other at the point of engagement but for the upper surface of the majority of the arms to lie in the same plane.

Figures 26 to 29 show the arrangement with the arms in the closed position. Here the knob 63 has been tightened so that the components of the ring are clamped together and a tooth 74 is located in a corresponding notch 75.

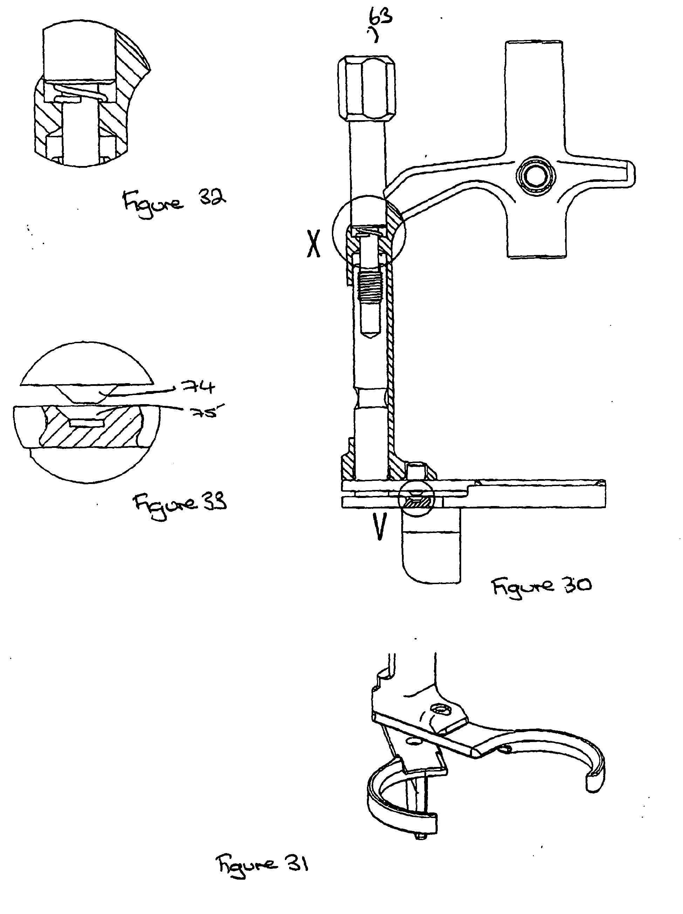

As shown in Figures 30 to 33, as the knob is loosened, the two arms forming the ring are allowed to move apart in a vertical direction such that the tooth 74 no longer engages the corresponding notch 75 and the arms can open.

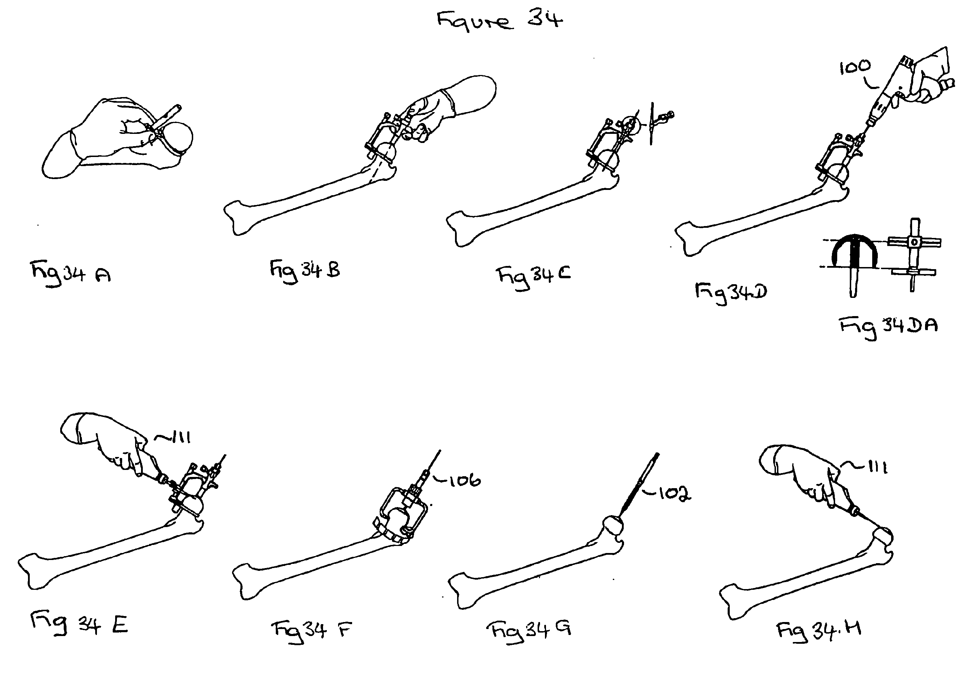

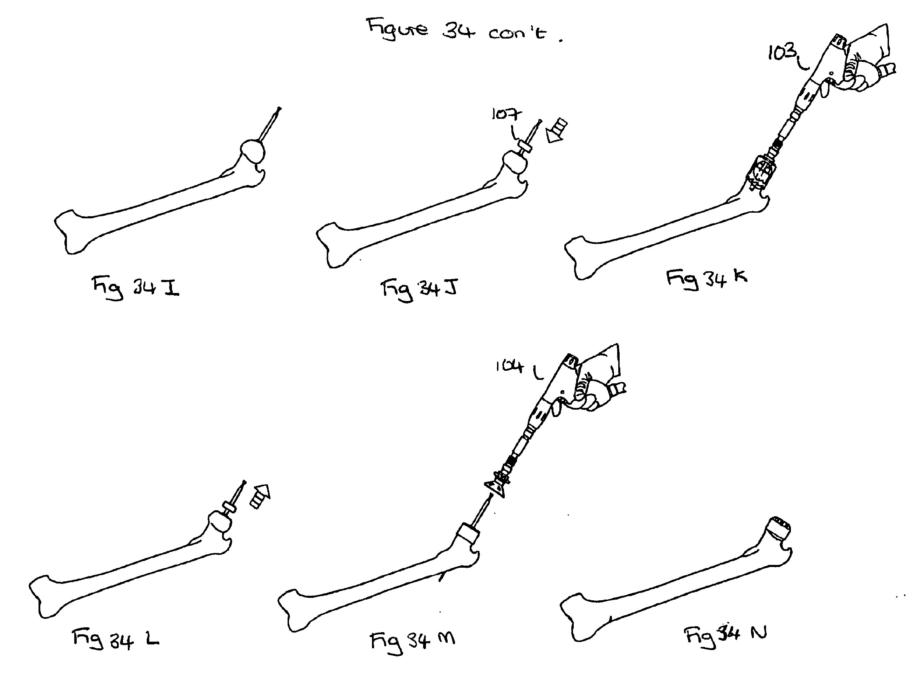

As illustrated in Figure 34, to use the arrangement as illustrated in Figures 17 to 33, the surgeon will first present one or more rings to the femur (Figure 34A) to select the appropriate size. Once the correct one is selected, the alignment guide of the present invention is assembled and the ring is located beneath the head of the femur at the headneck junction (Figure 34B). A guide wire if present is used to check the orientation (Figure 34C). A drill 100 is then inserted into the collar, optionally through the bore of the cannulated rod, and a well 101 is drilled in the head of the femur (Figure 34D). Figure 34DA illustrates the relationship between the ring and the saw guide of the alignment guide of the present invention and the base of the skirt of the prosthetic implant and the underside of the upper surface of the prosthetic implant.

The top of the head of the femur is then partially resected using the saw 111, the correct position of which is determined by the saw guide. In view of the relationship illustrated in Figure 34DA the cut will be the correct distance from the ring of the alignment guide (Figure 34E). It will be understood that it some arrangements, the top of the femur may be partially or fully resected before the well is drilled. The alignment guide is then removed and a check jig 106 may be used to assist the surgeon to satisfy himself that the size of sleeve cutter he has selected to machine the head of the femur, whilst machining the head to the required size will not impinge on the neck of the femur with the risk that it will be notched (Figure 34F). Any suitable check jig may be used but a particularly preferred arrangement is that described in EP 1634550.

If a guide rod 102 has been locate in the well it is removed (Figure 34G). If the top of the femur has only been partially resected it will then be fully removed (Figure 34H). The guide rod is then replaced (Figure 34I) and a spacer 107 is then passed over the guide rod 102 (Figure 34J).

This guide rod is then used to locate and correctly angle a sleeve cutter 103 (Figure 34K). The spacer 107 is then removed (Figure 34L) and a chamfering tool 104 is used. The chamfering tool 104 is located on the guide rod (Figure 34M). The femoral head is then ready (Figure 34N) to receive a femoral head resurfacing prosthesis (not shown).

It will be understood that the alignment guide of Figures 17 to 33 may be used in accordance with the method detailed above or that any of the alignment guides detailed above may be used following the sequence detailed here.

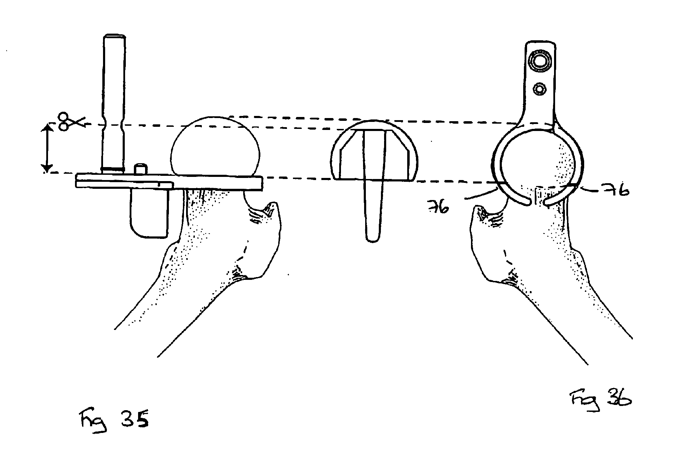

The relationship illustrated in Figure 34DA can be seen in more detail in Figure 35. A relationship between the external size of the ring and the external surface of the femoral head resurfacing prosthesis can be seen in Figure 36. In this arrangement, the external diameter of the ring has been formed to correspond to particular sizes of resurfacing prosthesis. Where the external surface of the ring is sized to correspond to the size of the prosthesis, when the alignment guide is in position, when the surgeon views the femoral head form above, he will be able to see the size of the prosthesis which is being used.

In this arrangement, the arms of the ring will have markings 76. The markings and the size mean that the surgeon can hold the ring against the femoral head and when the markings are position in the correct position for the skirt of the prosthesis, he can see whether the ring is the appropriate size for the head of the femur.

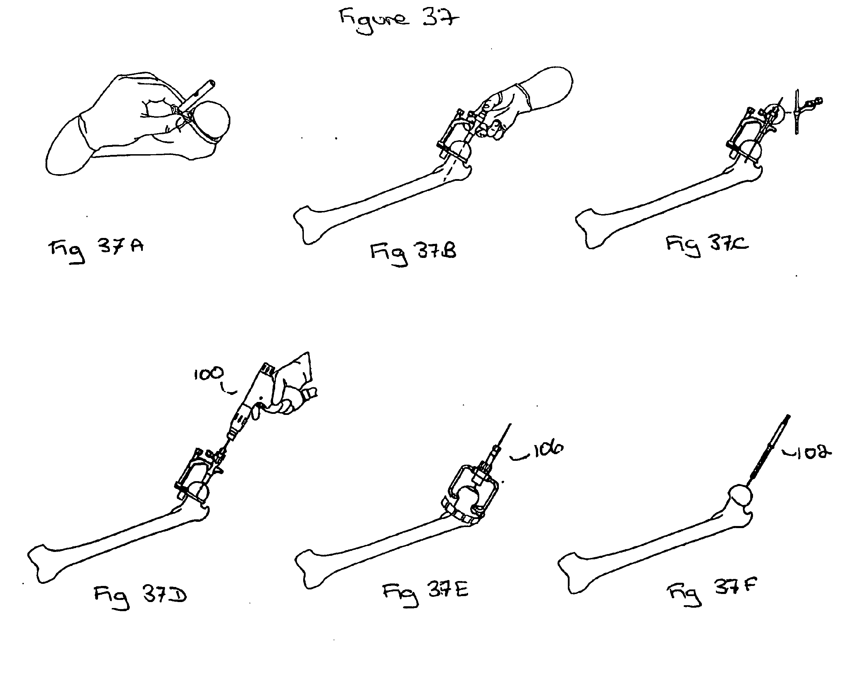

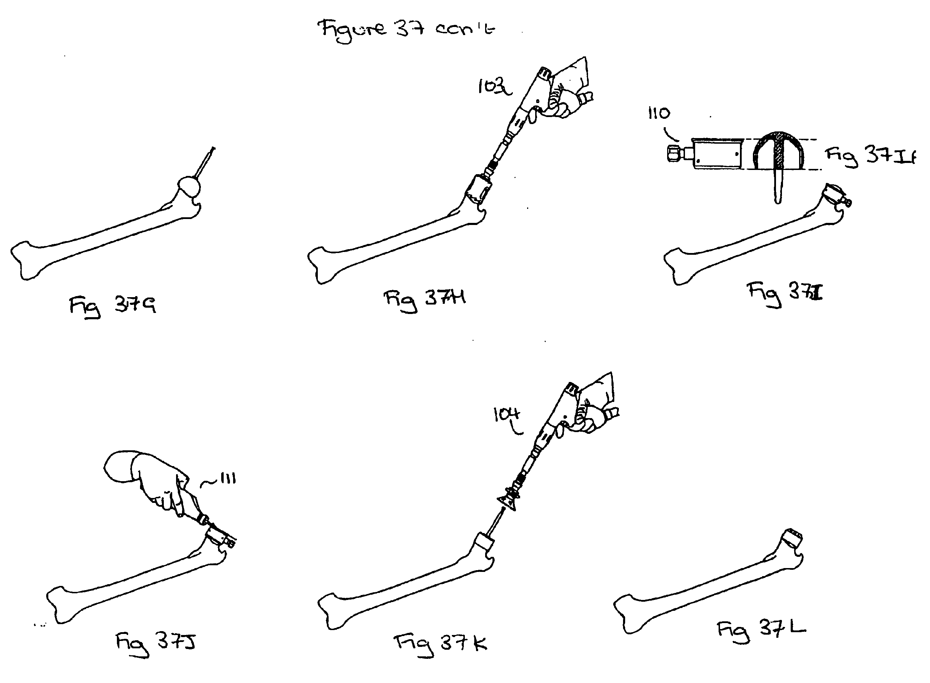

An alternative arrangement for use of the alignment guide of the present invention is described in Figure 37. This procedure is particularly suitable where the alignment guide does not include a saw cutting guide.

As illustrated in Figure 37, to use the arrangement as illustrated in Figures 17 to 33, the surgeon will first present one or more rings to the femur (Figure 37A) to select the appropriate size. Once the correct one is selected, the alignment guide of the present invention is assembled and the ring is located beneath the head of the femur at the headneck junction (Figure 37B). A guide wire if present is used to check the orientation (Figure 37C). A drill 100 is then inserted into the collar, optionally through the bore of the cannulated rod and a well is drilled in the head of the femur (Figure 34D).

The alignment guide is then removed and a check jig 106 may be used to assist the surgeon to satisfy himself that the size of sleeve cutter he has selected to machine the head of the femur, whilst machining the head to the required size will not impinge on the neck of the femur with the risk that it will be notched (Figure 37E). Any suitable check jig may be used but a particularly preferred arrangement is that described in EP1634550.

A guide rod 102 is then inserted into the well (Figure 37F) and is shown in position in Figure 37G. This guide rod is then used to locate and correctly angle a sleeve cutter 103 (Figure 37H).

A separate saw guide 110 is then located over the cut head (Figure 37I). Figure 37IA illustrates the relationship between the separate saw guide and the base of the skirt of the prosthetic implant and the underside of the upper surface of the prosthetic implant.

The top of the head of the femur is then removed using the saw 111 guided by the separate saw guide. In view of the relationship illustrated in Figure 37IA the cut will be in the correct position. It will be understood that it some arrangements, the top of the femur may be removed before the well is drilled.

The separate saw guide 107 is then removed and a chamfering tool 104 is used. The chamfering tool is located on the guide rod (Figure 37K). The femoral head is then ready (Figure 34L) to receive a femoral head resurfacing prosthesis (not shown).

It will be understood that the alignment guide of Figures 17 to 33 may be used in accordance with this method or that any of the alignment guides of the present invention which do not include a saw guide may be used following this sequence.

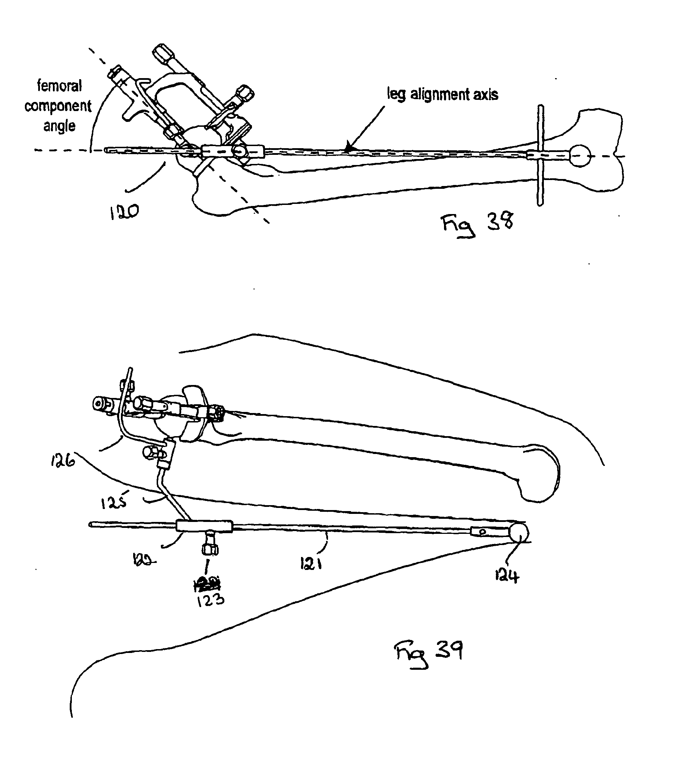

The alignment guide of the present invention may be used in combination with an elongate distal alignment guide 120. The elongate distal alignment guide 120 is illustrated in Figures 38 to 40. The elongate distal alignment guide is used to suggest an optimum femoral component angle for the resurfacing head implant. The alignment guide of the present invention suggests an angle for insertion of the guide wire and ultimately the final implanted femoral resurfacing head prosthesis, relative to the leg alignment axis. The leg alignment axis is a theoretical line between the centre of the femoral head, middle of the knee and middle of the ankle when the person is standing. The axis can be measured easily between the femoral head and knee on a patient in surgery. The position of the axis and the femoral component angle are illustrated in Figure 38.

The elongate distal alignment guide may be attached to the alignment guide of the present invention or it may be a separate component which may be connectable to the alignment guide of the present invention or separate therefrom. Where it is separate, it will touch on the elongate guide of the present invention to measure the current femoral component angle.

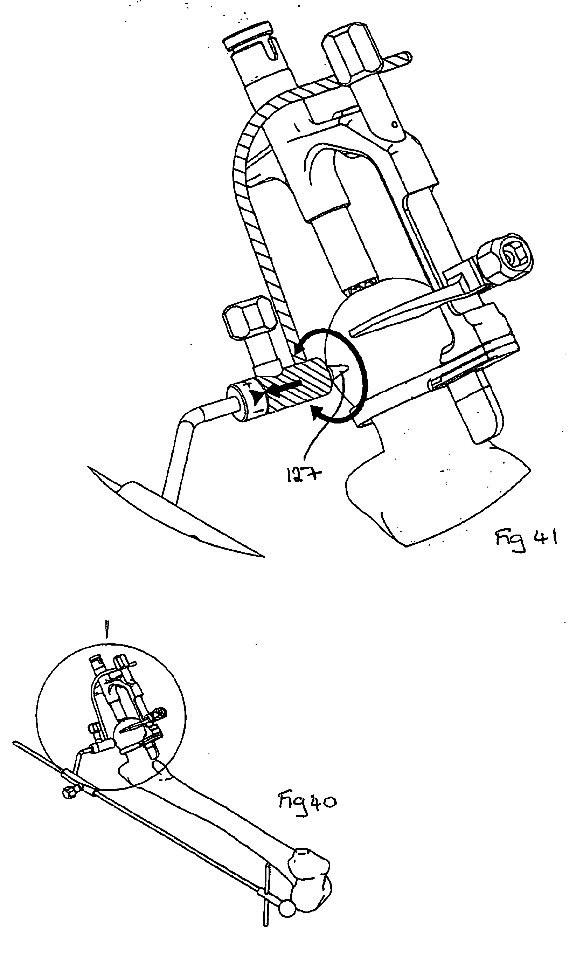

As illustrated in Figure 39, whether the elongate alignment guide is fixed or separate it will generally comprise a long rod 121 slidable in a collar 122 so that its length can be adjusted. A locking nut 123 will be provided to lock the rod at the desired length. A stop 124 may be provided at the end of the rod which in use will come into contact with the back of the patient's knee to protect the skin. Arms 125, 126 extend from the collar 122 and enable the rod to be either fixed to the alignment guide of the present invention or impinge therewith. Arm 126 may be referred to as a probe arm. The angle between the rod 121 and the probe arm 126 may be fixed or it may be adjustable. An adjustable arrangement is illustrated in more detail in Figures 40 and 41. In Figure 41, the "+" sign implies that the alignment guide of the present invention needs to be placed more in valgus and the "-" implies that it needs to be placed in more varus. The area shown hatched in Figure 41 is the area that is free to rotate on the shaft until it touches on the alignment guide of the present invention. In an alternate arrangement, the "+/-" display may be placed with a display showing degrees.

In use, the spiked end 127 should be placed at the centre of the femoral head as this represents the start of the leg alignment axis.

It will be understood that the elongate distal alignment guide described above may be used with any alignment guide of the present invention or any other alignment guide.

## Claims

1. An alignment guide (20; 20'; 20") for use in femoral head surgery comprising:
a support arm (-; 32; 32') having a proximal (23; 23'; 23") and a distal (22; 22'; 22") end;
a ring (24; 24'; 24") located at the distal end of the support arm and angled to the plane extending along the support arm;
a collar (26; 26'; 26") located at the proximal end of the support arm; and a cannulated rod (25; 25'; 25"); said rod being a sliding fit in the collar (26; 26'; 26") and having teeth (28; 28'; 28") located at the end thereof which in use will come into contact with the femoral head
the support arm being configured such that the axis of the bore of the collar passes through the centre point of the ring.

2. An alignment guide according to Claim 1 wherein the ring ( 24; 24'; 24") is angled at between 80° and 100° to the plane extending along the support arm.

3. An alignment guide according to Claim 1 wherein the ring is substantially perpendicular to the plane of the support arm.

4. An alignment guide according to any one of Claims 1 to 3 wherein the ring is adjustable such that the centre of the ring can be displaced from the axis of the bore.

5. The alignment guide according to any one of Claims 1 to 4 wherein a locking means is provided to lock the rod in position.

6. The alignment guide according to Claim 5 wherein the locking means is a locking screw (27; 27'; 27").

7. The alignment guide according to any one of Claims 1 to 6 wherein the cannulated rod additionally includes measuring indica.

8. The alignment guide according to any one of Claims 1 to 7 wherein the outside edge of the ring is sized to replicate the maximum size of a femoral head.

9. The alignment guide according to Claim 8 wherein the ring includes markings which when the ring is used as a size checkit represents the position of the skirt of a prosthesis.

10. The alignment guide according to any one of Claims 1 to 9 wherein the ring is a C-ring.

11. The alignment guide according to any one of Claims 1 to 9 wherein the ring is an expandable iris.

12. The alignment guide according to any one of Claims 1 to 11 wherein the ring includes a removable portion, said removable portion being hinged to the remainder of the ring and moveable from an open to a closed position.

13. The alignment guide according to any one of Claims 1 to 12 wherein the ring is formed of two arms which are each hinged to allow movement from an open to a closed position.

14. The alignment guide according to Claim 13 wherein when in the closed position, the arms complete a circle.

15. The alignment guide according to any one of Claims 1 to 11 wherein the ring is formed of two arms one of which is fixed and the other of which is movable from an open to a closed position.

16. The alignment guide according to any one of Claims 1 to 15 wherein the collar has a slot extending along at least a portion thereof.

17. The alignment guide according to any one of Claims 1 to 16 wherein a flag is included at the distal end of the support arm and extends below the position of the ring.

18. The alignment guide according to any one of Claims 1 to 17 wherein in use the ring replicates the rim of a resurfacing head prosthesis.

19. The alignment guide according to any one of Claims 1 to 18 additionally includes a saw cutting guide (33) mounted on the support arm (32), collar or, where present, on the cannulated rod.

20. The alignment guide according to any one of Claims 1 to 19 wherein the ring is demountable.

21. The alignment guide according to any one of Claims 1 to 20 in combination with an elongate distal alignment guide which by referencing the back of the knee will reference the leg alignment axis..

22. A kit comprising the alignment guide of Claim 21 and a plurality of rings.

23. A kit according to Claim 22 wherein the plurality of rings are of differing sizes.

24. A kit according to Claim 22 or 23 wherein the kit additionally includes an elongate distal alignment guide which by referencing the back of the knee will reference the leg alignment axis.

## Patentansprüche

1. Ausrichtführung (20;20';20") zur Benutzung bei der Femurkopfchirurgie mit
einem Tragarm (-;32;32') mit einem proximalen (23;23';23") und einem distalen (22;22';22") Ende,
einem Ring (24;24';24"), der an dem distalen Ende des Tragarms und winklig zu der sich längs des Tragarms erstreckenden Ebene angeordnet ist,
einer Manschette (26;26';26"), die an dem proximalen Ende des Tragarms angeordnet ist, und einem röhrenförmigen Stab (25;25';25"), der mit Gleitsitz in der Manschette (26;26';26") ist und an seinem Ende angeordnete Zähne (28;28';28") hat, die bei der Benutzung mit dem Femurkopf in Berührung kommen, wobei der Tragarm so ausgebildet ist, daß die Achse der Bohrung der Manschette durch den Mittelpunkt des Rings verläuft.

2. Ausrichtführung nach Anspruch 1, bei der der Ring (24;24';24") zwischen 80° und 100° zu der sich längs des Tragarms erstreckenden Ebene abgewinkelt ist.

3. Ausrichtführung nach Anspruch 1, bei der der Ring im wesentlichen senkrecht zu der Ebene des Tragarms ist.

4. Ausrichtführung nach einem der Ansprüche 1 bis 3, bei der der Ring so einstellbar ist, daß die Mitte des Rings aus der Achse der Bohrung verschoben werden kann.

5. Ausrichtführung nach einem der Ansprüche 1 bis 4, bei der ein Feststellmittel zur Positionsfestlegung des Stabes vorgesehen ist.

6. Ausrichtführung nach Anspruch 5, bei der das Feststellmittel eine Feststellschraube (27;27';27") ist.

7. Ausrichtführung nach einem der Ansprüche 1 bis 6, bei der der röhrenförmige Stab zusätzlich Meßmarken hat.

8. Ausrichtführung nach einem der Ansprüche 1 bis 7, bei der der Außenrand des Rings größenmäßig die Maximalgröße eines Femurkopfes nachbildet.

9. Ausrichtführung nach Anspruch 8, bei der der Ring Markierungen trägt, die bei Benutzung des Rings als Größenprobe die Position des Randes der Prothese darstellt.

10. Ausrichtführung nach einem der Ansprüche 1 bis 9, bei der der Ring ein C-Ring ist.

11. Ausrichtführung nach einem der Ansprüche 1 bis 9, bei der der Ring eine expandierbare Blende ist.

12. Ausrichtführung nach einem der Ansprüche 1 bis 11, bei der der Ring einen entfernbaren Teil hat, der an dem Rest des Ringes gelenkig angebracht und aus einer offenen in eine geschlossene Position bewegbar ist.

13. Ausrichtführung nach einem der Ansprüche 1 bis 12, bei der der Ring aus zwei Armen gebildet ist, die beide gelenkig angebracht sind, um eine Bewegung aus einer offenen in eine geschlossene Position zu ermöglichen.

14. Ausrichtführung nach Anspruch 13, bei der die Arme in der geschlossenen Position einen vollständige Kreis bilden.

15. Ausrichtführung nach einem der Ansprüche 1 bis 11, bei der der Ring aus zwei Armen gebildet ist, von denen einer fest angeordnet ist und der andere von einer offenen in eine geschlossene Position beweglich ist.

16. Ausrichtführung nach einem der Ansprüche 1 bis 15, bei der die Manschette einen längs wenigstens eines Teils von ihr verlaufende Schlitz hat.

17. Ausrichtführung nach einem der Ansprüche 1 bis 16, bei der eine Positionsfahne an dem distalen Ende des Tragarms angeordnet ist und sich unter die Position des Rings erstreckt.

18. Ausrichtführung nach einem der Ansprüche 1 bis 17, bei der bei Benutzung der Ring den Rand einer die Oberfläche wieder herstellenden Kopfprothese nachbildet.

19. Ausrichtführung nach einem der Ansprüche 1 bis 18, ferner mit einer Sägesrhnittführung (33), die auf dem Tragarm (32), der Manschette oder, wo vorhanden, auf dem röhrenförmigen Stab angebracht ist.

20. Ausrichtführung nach einem der Ansprüche 1 bis 19, bei der der Ring abmontierbar ist.

21. Ausrichtführung nach einem der Ansprüche 1 bis 20 in Verbindung mit einer länglichen distalen Ausrichtführung, die durch Bezug auf die Rückseite des Knies auf die Beinausrichtungsachse Bezug nimmt.

22. Instrumentesatz mit der Ausrichtführung des Anspruchs 21 und einer Mehrzahl von Ringen.

23. Instrumentesatz nach Anspruch 22, bei dem die Mehrzahl der Ringe unterschiedliche Größen hat.

24. Instrumentesatz nach Anspruch 22 oder 23, der zusätzlich eine längliche distale Ausrichtführung hat, die durch Bezug auf die Rückseite des Knies auf die Beinausrichtungsachse Bezug nimmt.

## Revendications

1. Guide d'alignement (20 ; 20' ; 20") pour utilisation dans la chirurgie de la tête de fémur comprenant :
un bras de support (- ; 32 ; 32') ayant une extrémité proximale (23 ; 23' ; 23") et une extrémité distale (22 ; 22' ; 22") ;
un anneau (24 ; 24' ; 24") situé à l'extrémité distale du bras de support et incliné par rapport au plan s'étendant le long du bras de support ;
un collier (26 ; 26' ; 26") situé à l'extrémité proximale du bras de support ; et
une tige tubulaire (25 ; 25' ; 25"); ladite tige s'emmanchant de façon coulissante dans le collier (26 ; 26' ; 26") et ayant des dents (28 ; 28' ; 28") situées à l'extrémité qui, pendant l'utilisation, entreront en contact avec la tête de fémur,
le bras de support étant configuré de telle sorte que l'axe de l'alésage du collier passe à travers le point central de l'anneau.

2. Guide d'alignement selon la revendication 1, dans lequel l'anneau (24 ; 24' ; 24") est incliné à un angle compris entre 80° et 100° par rapport au plan s'étendant le long du bras de support.

3. Guide d'alignement selon la revendication 1, dans lequel l'anneau est sensiblement perpendiculaire au plan du bras de support.

4. Guide d'alignement selon l'une quelconque des revendications 1 à 3, dans lequel l'anneau est ajustable de telle sorte que le centre de l'anneau puisse être déplacé par rapport à l'axe de l'alésage.

5. Guide d'alignement selon l'une quelconque des revendications 1 à 4, dans lequel des moyens de verrouillage sont prévus pour verrouiller la tige en position.

6. Guide d'alignement selon la revendication 5, dans lequel les moyens de verrouillage sont une vis de verrouillage (27 ; 27' ; 27").

7. Guide d'alignement selon l'une quelconque des revendications 1 à 6, dans lequel la tige tubulaire comprend des repères de mesure.

8. Guide d'alignement selon l'une quelconque des revendications 1 à 7, dans lequel le bord extérieur de l'anneau est dimensionné pour reproduire la taille maximale d'une tête de fémur.

9. Guide d'alignement selon la revendication 8, dans lequel l'anneau comprend des marquages qui, lorsque l'anneau est utilisé en tant qu'élément de contrôle de dimension, représentent la position de la jupe d'une prothèse.

10. Guide d'alignement selon l'une quelconque des revendications 1 à 9, dans lequel l'anneau est un anneau en C.

11. Guide d'alignement selon l'une quelconque des revendications 1 à 9, dans lequel l'anneau est un iris extensible.

12. Guide d'alignement selon l'une quelconque des revendications 1 à 11, dans lequel l'anneau comprend une partie amovible, ladite partie amovible étant articulée sur le reste de l'anneau et pouvant être déplacée d'une position ouverte à une position fermée.

13. Guide d'alignement selon l'une quelconque des revendications 1 à 12, dans lequel l'anneau est formé de deux bras qui sont chacun articulés pour permettre le mouvement d'une position ouverte à une position fermée.

14. Guide d'alignement selon la revendication 13, dans lequel lorsqu'il est dans la position fermée, les bras réalisent un cercle.

15. Guide d'alignement selon l'une quelconque des revendications 1 à 11, dans lequel l'anneau est formé de deux bras dont l'un est fixe et l'autre peut déplacé d'une position ouverte à une position fermée.

16. Guide d'alignement selon l'une quelconque des revendications 1 à 15, dans lequel le collier a une fente s'étendant le long d'au moins une partie de celui-ci.

17. Guide d'alignement selon l'une quelconque des revendications 1 à 16, dans lequel un indicateur est inclus à l'extrémité distale du bras de support et s'étend sous la position de l'anneau.

18. Guide d'alignement selon l'une quelconque des revendications 1 à 17, dans lequel, en cours d'utilisation, l'anneau reproduit le bord d'une prothèse de tête de resurfaçage.

19. Guide d'alignement selon l'une quelconque des revendications 1 à 18, qui comprend en outre un guide de coupe à la scie (33) monté sur le bras de support (32), le collier ou, le cas échéant, sur la tige tubulaire.

20. Guide d'alignement selon l'une quelconque des revendications 1 à 19, dans lequel l'anneau est démontable.

21. Guide d'alignement selon l'une quelconque des revendications 1 à 20, en combinaison avec un guide d'alignement distal allongé qui, en désignant l'arrière du genou, désignera l'axe d'alignement de la jambe.

22. Kit comprenant le guide d'alignement selon la revendication 21 et une pluralité d'anneaux.

23. Kit selon la revendication 22, dans lequel la pluralité d'anneaux sont de taille différente.

24. Kit selon la revendication 22 ou 23, dans lequel le kit comprend en outre un guide d'alignement distal allongé qui, en désignant l'arrière du genou, désignera l'axe d'alignement de la jambe.
